# EUROPEAN PATENT APPLICATION

(11) **EP 1 170 375 A2**
(43) Date of publication of application: **09.01.2002**
(21) Application number: 01305536.3
(22) Date of filing: 26.06.2001
(51) Int. Cl.: C12P 7/22, C12P 7/44

(54) **Microbial transformation process for hydroxyaromatic compounds**

(30) Priority: 27.06.2000 JP 2000193131
(71) Applicant: NISSAN CHEMICAL INDUSTRIES LTD., Chiyoda-ku Tokyo (JP)
(72) Inventor: Nagasawa, Toru, Gifu-shi, Gifu (JP); Yoshida, Toyokazu, Gifu-shi, Gifu (JP); Takigawa, Shinichiro, Nissan Chemical Ind. Limited, Chiyoda-ku, Tokyo (JP); Suzuki, Hideo, Nissan Chemical Ind. Limited, Funabashi-shi, Chiba (JP)
(74) Representative: Eddowes, Simon

(57) **Abstract**

The present invention provides a microbial transformation process for producing a hydroxyaromatic compound represented by the formula (II): 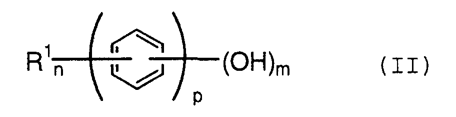 wherein R¹ represents carboxyl group, C₂₋₅ alkoxycarbonyl group, hydroxyl group, nitro group, cyano group or C₁₋₅ alkyl group, m is an integer of 1 to 5, n is an integer of 0 to 5, p is an integer of 1 to 3, m, n and p satisfy a relation of m+n≦4+2p, and when n is 2 or more, R¹ may be the same or different from each other,
which comprises bringing an aromatic sulfonic acid compound represented by the formula (I) : 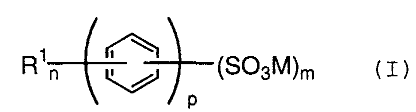 wherein each of R¹, m, n and p has the same meaning as defined above, and M represents hydrogen, alkali metal, alkaline earth metal or C₁₋₅ alkyl group;
in contact with a culture medium, cells, or treated cells of a microorganism belonging to *Ochrobactrum* genus, and
*Ochrobactrum anthropi* S9 deposited under a receipt number of FERM BP-7546.

## Description

### BACKGROUND OF THE INVENTION

### 1. Field of the invention

This invention relates to a microbial translformation process for producing hydroxyaromatic compounds.

### 2. Prior art

Hydroxyaromatic compounds have been conventionally produced by means of synthetic chemistry. For example, a method is generally employed in which an aromatic compound is sulfonated followed by a reaction with alkali under a high temperature condition, so-called an alkali-fusion, thereby introducing a hydroxyl group. However, in this method, disposal of a large amount of an alkaline waste fluid is required, and therefore, industrial production process that is more effective has been sought.

On the other hand, there has not been reported a method of converting a sulfone group to a hydroxyl group by means of microorganisms.

### SUMMARY OF THE INVENTION

An object of the present invention is to provide a microbial transformation process for producing hydroxyaromatic compounds that satisfies the need described above.

The present inventors have made extensive and intensive studies and found that a microorganism *Ochrobactrum anthropi* S9 that belongs to *Ochrobactrum* genus transforms an aromatic sulfonic acid compound to a hydroxyaromatic compound, and thus, the present invention has been completed.

Accordingly, the present invention relates to a microbial transformation process for producing a hydroxy aromatic compound represented by the formula (II): 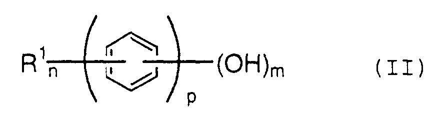 wherein R¹ represents a carboxyl group, a C₂₋₅ alkoxycarbonyl group, a hydroxyl group, a nitro group, a cyano group or a C₁₋₅ alkyl group, m represents an integer of 1 to 5, n represents an integer of 0 to 5, p represents an integer of 1 to 3, m, n and p satisfy a relation of m+n≦4+2p, and when n is 2 or more, R¹ may be the same or different from each other,
which comprises bringing an aromatic sulfonic acid compound represented by the formula (I) 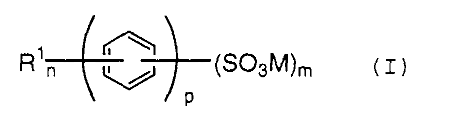 wherein each of R¹, m, n and p has the same meaning as defined above, and M represents a hydrogen atom, an alkali metal, an alkaline earth metal or a C₁₋₅ alkyl group,
in contact with a culture medium, cells or treated cells of a microorganism belonging to *Ochrobactrum* genus.

Further, the present invention relates to a microorganism belonging to *Ochrobactrum* genus of *Ochrobactrum* anthropi S9 which is deposited at the International Patent Organism Depository; National Institute of Advanced Industrial Science and Technology, Japan under a receipt number of FERM P-17918 which corresponds to FERM BP-7546 having an activity of hydroxylating an aromatic sulfonic acid compound.

### DESCRIPTION OF THE PREFERRED EMBODIMENTS

Hereinbelow, the present invention will be explained in more detail.

The compound represented by the formulae (I) and (II) regarding the present invention are explained hereinafter.

R¹ in the formula (I) and (II) represents a carboxyl group, C₂₋₅ alkoxycarbonyl group, a hydroxyl group, a nitro group, a cyano group or C₁₋₅ alkyl group.

In the description hereinbelow, n represents normal, i represents iso, c represents cyclo, s represents secondary and t represents tertiary.

As the C₂₋₅ alkoxycarbonyl group, there may be mentioned methoxycarbonyl group, ethoxycarbonyl group, n-propoxycarbonyl group, i-propoxycarbonyl group, c-propoxycarbonyl group, n-butoxycarbonyl group, i-butoxycarbonyl group, s-butoxycarbonyl group, t-butoxycarbonyl group, c-butoxycarbonyl group, 1-methyl-c-propoxycarbonyl group and 2-methyl-c-propoxycarbonyl group and the like.

Among them, methoxycarbonyl group, ethoxycarbonyl group, n-propoxycarbonyl group, i-propoxycarbonyl group, c-propoxycarbonyl group and the like are preferable, and methoxycarbonyl group is more preferable.

As the C₁₋₅ alkyl group, there may be mentioned methyl group, ethyl group, n-propyl group, i-propyl group, c-propyl group, n-butyl group, i-butyl group, s-butyl group, t-butyl group, c-butyl group, 1-methyl-c-propyl group, 2-methyl-c-propyl group, n-pentyl group, 1-methyl-n-butyl group, 2-methyl-n-butyl group, 3-methyl-n-butyl group, 1,1-dimethyl-n-propyl group, 1,2-dimethyl-n-propyl group, 2,2-dimethyl-n-propyl group, 1-ethyl-n-propyl group, c-pentyl group, 1-methyl-c-butyl group, 2-methyl-c-butyl group, 3-methyl-c-butyl group, 1,2-dimethyl-c-propyl group, 2,3-dimethyl-c-propyl group, 1-ethyl-c-propyl group, 2-ethyl-c-propyl group and the like.

As preferred, methyl group, ethyl group, n-propyl group, i-propyl group, c-propyl group, etc. are listed.

M in the formula (I) represents a hydrogen atom, an alkali metal, an alkaline earth metal or a C₁₋₅ alkyl group.

As the alkali metal, there may be mentioned lithium, potassium, sodium and the like, among which sodium is preferred.

As the alkaline earth metal, there may be mentioned beryllium, magnesium, calcium, strontium, barium and the like, among which magnesium and calcium are preferred.

As the C₁₋₅ alkyl group, the same as the above described groups are mentioned, preferably methyl group, ethyl group, n-propyl group, i-propyl group and c-propyl group.

m is a number of SO₃M, representing an integer of 1 to 5, preferably 1 to 3, and more preferably 1.

n is a number of R¹, representing an integer of 0 to 5, preferably 1 or 2. When n is 2 or more, R¹ may be the same or different from each other.

p is a number of a benzene ring, representing an integer of 1 to 3. Preferred are 1 to 3 and more preferably 1. That is, there may be mentioned as an aromatic compound, benzene ring, naphthalene ring, anthracene ring and phenanthrene ring, among which benzene ring is preferred.

m, n and p satisfy a relation of m+n≦4+2p

Specific examples of the compound represented by the formula (I) may include, for example, benzenesulfonic acid sodium salt, 1-naphthalenesulfonic acid sodium salt and 5-sulfonaphthalic acid sodium salt.

The preferred compounds regarding the present invention are as described below.
1. A compound in which R¹ is a carboxyl group or a C₂₋₅ alkoxycarbonyl group, and p is 1.
2. The above mentioned compound in which n is 2 and m is 1.
3. Examples of more preferred compounds include a case in which the compound represented by the formula (I) is 5-sulfoisophthalic acid sodium salt and the compound represented by the formula (II) is 5-hydroxyisophthalic acid as mentioned below. 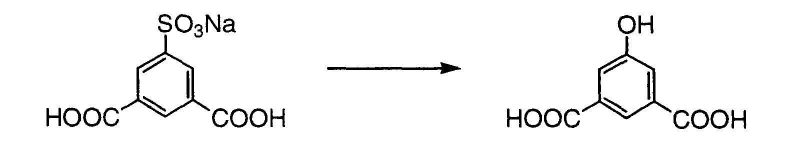
As a microorganism to be used in the present invention that belongs to *Ochrobactrum* genus, there can be mentioned a bacteria strain named *Ochrobactrum anthropi* S9 (accession number FERM P-17918 which is an international deposition number FERM BP-7546).

Mycological properties of the present bacteria strain are as described below.
Morphological properties;
   Shape and size of the cell: bacillus with a size of 0.72 x 1 to 1.2 µm
   Gram stain: (-)
   Flagella attachment status: lateral
   Presence of spore: none
Culture relating properties;
   Bouillon agar plate culture
   Shape of colony: circular
   Form of peripheral part of colony: entire fringe
   Form of surface of colony: smooth
   Color of colony: pale yellow to opalescent
Physiological properties;
   Denitrification reaction: (+)
   MR test: (-)
   Indole synthesis: (-)
   Hydrogen sulfide synthesis: (-)
   Hydrolysis of starch: (-)
   Reduction of nitrate: (+)
   Use of citric acid:
      1) Koser: (+)
      2) Christensen: (+)
   Use of inorganic nitrogen source:
      1) NaNO₃: (+)
      2) (NH₄)₂SO₄: (+)
   Urease: (+)
   Oxidase: (+)
   Catalase: (+)
   Arginine dihydrolase: (-)
   Gelatin decomposition: (-)
   Esculin hydrolysis: (-)
   β-galactosidase: (-)
   Temperature range for culture: room temperature to 37°C
   Attitude toward oxygen: facultative anaerobic
   O-F test: (-)
Ability to utilize substrate;
   Glucose: (+)
   L-arabinose: (+)
   D-mannose: (+)
   D-mannitol: (+)
   N-acetyl-D-glucosamine: (+)
   Maltose: (-)
   Potassium gluconate: (-)
   n-capric acid: (-)
   Adipic acid: (-)
   dl-malic acid: (+)
   Sodium citrate: (-)
   Phenyl acetate: (-)
Synthesis of acid or gas from sugars (acid/gas)
   L-arabinose: (+/-)
   D-glucose: (-/-)
   D-fructose: (-/-)
   Maltose: (-/-)
   Lactose: (-/-)
   D-sorbitol: (-/-)
   Inositol: (-/-)
   Starch: (-/-)
   D-xylose: (+/-)
   D-mannose: (-/-)
   D-galactose: (-/-)
   Sucrose: (-/-)
   Trehalose: (-/-)
   D-mannitol: (-/-)
   Glycerin: (+/-)

Based on the above, the present bacteria strain is identified as *Ochrobackrum anthropi* S9 strain.

The present invention can be conducted in various embodiments. In this case, for a purpose of convenience in treating, microorganisms are used in a state of dried cells such as freeze-dried cells, spray-dried cells and the like, as well as a treated cells such as treated cells with acetone or toluene and the like or disrupted cells, cell extract, etc. For example, there can be mentioned:
(a) a method wherein microorganisms are cultured in a culture medium containing the compound represented by the formula (1) as a substrate;
(b) a method wherein the microorganisms are collected from a culture medium where they were cultured and brought into contact with the compound represented by the formula (1); and
(c) a method wherein a cell-free extract prepared from the cells of the microorganisms are brought into contact with the compound represented by the formula (1) and so on.

Culture of the transforming microorganism of the present invention is conducted in a culture media containing a nutrient that can be generally utilized by the microorganisms. As a nutrient source, the known nutrients used for culturing general microorganisms can be used.

For example, as a source of carbon, glucose, fructose, maltose, sucrose, mannitol, sodium glutamate, glycerol, succinic acid, dextrin, oat, rye, cornstarch, potato, glycerin and the like are used.

As a source of nitrogen, soybean powder, wheat germ, meat powder, fishpowder, bouillon, peptone, corn steep liquor, dried yeast, an ammonium salt such as ammonium nitrate, and the like are used.

Besides those, as necessity arises, additives can be used in combination, which helps growth of the microorganisms and enhances a synthesis of an enzyme having a hydroxylation activity to be utilized in the present invention, as well as a salt of inorganic material such as sodium chloride, potassium chloride, calcium carbonate, phosphates and the like.

In use of liquid culture, silicone oil, a vegetable oil, a surfactant, etc. are used as an anti-foaming agent.

Culture is conducted under an aerobic condition, and generally used aerobic culture methods are, for example, solid culture, shaking culture, aeration-agitation culture and the like.

A temperature used for culture is in the range of 20 to 30°C, preferably in the range of 25 to 30°C.

The method (a) is carried out by culturing the microorganisms with addition of a compound represented by the formula (1). Timing of adding the compound depends on a suitable culture conditions for the transforming microorganisms to be used, especially, culture apparatus, culture composition, culture temperature and the like, it is preferably added at a point where an hydroxylation activity of the transforming microorganisms starts to elevate. Generally, it is preferable to add the compound 1 to 5 days after the start of culturing the transforming microorganisms. An amount added of the starting compound, that is, a substrate, is in the range of 0.01 to 5.0% based on the medium, preferably in the range of 0.025 to 0.5%.

After the addition of the starting compound, culture is carried out under an aerobic condition at the above-mentioned temperature. Duration of culture is generally 1 to 8 days after the addition of the starting compound.

The method (b) is carried out by culturing the transforming microorganisms in the presence of a little amount of a substrate by the above-mentioned method (a), until an hydroxylation activity of the transforming microorganisms is maximized.

That is, though an hydroxylation activity depends on a type of a culture medium, a temperature and so on, it is generally maximized 2 or 3 days after the start of culture, thus, the culture is terminated at that point. The transforming microorganisms are collected by centrifugal separation, filtration, etc. Generally, it is preferable that the collected cells of the transforming microorganisms are used after being washed with physiological saline, buffer and the like. When the thus obtained transforming microorganisms are brought into contact with a starting compound, it is generally carried out in an aqueous solvent, for example, in a 10 to 100 mM phosphate buffer of pH 5 to 9. A reaction due to contact is carried out normally at a temperature of 20 to 33°C, preferably 25 to 30°C. A concentration of the substrate is normally in the range of 0.01 to 5.0% based on the culture medium. A reaction time depends on a concentration of the substrate, a reaction temperature, and the like, and it is generally 1 to 8 days.

The cell-free extract used in the method (c) is obtained by applying a physical or chemical means to the microorganisms obtained in the above-mentioned method, for example, in a form of crushed cells by trituration, ultrasonic treatment, etc. or in a form of a dissolved solution of the cells by treating with an organic solvent such as acetone, toluene and the like, a surfactant, an enzyme, etc.

When the cell-free extract, that is the treated microorganism thus obtained, is brought into contact with a starting compound, it is carried out in the same manner as in the above-described method of bringing microorganisms into contact with the starting compound.

Further, any method can be used for a reaction which is employed as an enzyme reaction system such as a method in which those enzymes and enzyme containing substances are immobilized by a conventional manner, etc.

After completion of the conversion reaction, the objective compound can be collected, separated and purified by the conventionally known method from products. For example, the obtained product is extracted by using an organic solvent that is difficultly miscible with water such as ethyl acetate or butanol under an acidic condition, and then, after the solvent is distilled from the extract, the obtained crude objective compound is subjected to a column chromatography using silica gel, alumina and the like and the objective compound can be separated and purified by elution using a proper eluent. Or else, the extract in the organic solvent is dissolved in a basic aqueous solution such as an aqueous alkaline solution, subsequently by means of a column chromatography using anion exchange resins, the product can be separated and purified. In addition, the product of a high concentration can give a crystal in an aqueous solution.

As a process for producing a hydroxy aromatic compound, the microbial method of the present invention for converting a sulfone group to a hydroxyl group enables to inhibit formation of byproducts under mild conditions, without a need for a complicated separation procedure, thereby making it possible to advantageously produce a hydroxy aromatic compound.

### EXAMPLE

Hereinbelow, the present invention will be described in more detail with reference to the following Examples, which should not be construed as limiting the scope of the present invention.

### Reference Example 1

### Separation of 5-hydroxyisophthalic acid from converted products.

In a culture medium containing converted products, 3 mol /L of hydrochloric acid was added to adjust pH to 1.0, then, the same amount (volume) of butanol as the culture medium was added and extracted. To the butanol layer, the same amount (volume) of 0.1 M sodium hydrogen carbonate/sodium hydroxide (pH 9.0) was added for extraction. An aqueous layer was subjected to an anion exchange resin column comprising Dowex (trade name, available from Dow Chemical) 1 x 2, OH⁻ type, then eluted with 2 mol/L of acetic acid to thereby isolate 5-hydroxyisophthalic acid.

### Reference Example 2

### Determination of 5-hydroxyisophthalic acid

An amount of 5-hydroxyisophthalic acid was determined by high performance liquid chromatography (HPLC) under the condition described below.
Column: ODS column (Waters Co., Trade name: Spherisorb S50DS2, 4.6x150mm)
Eluent: 73 mM KH₂PO₄-H₃PO₄ (pH 3.0)/acetonitrile = 92:8
Flow rate: 1.0 mL/min
Detection: ultraviolet absorption at 230 nm

### Example 1

Culture medium is prepared as shown in Table 1 to 3.

**Table 1**

| Composition of the culture medium | |
|---|---|
| Glucose | 20.0 g |
| Ammonium chloride | 2.0 g |
| Sodium glutamate | 15.0 g |
| Potassium dihydrogen phosphate | 0.5 g |
| Dipotassium hydrogen phosphate | 4.0 g |
| Magnesium chloride hexahydrate | 0.2 g |
| Magnesium chloride dihydrate | 0.01 g |
| Sodium chloride | 0.01 g |
| Metal solution | 10 mL |
| Vitamin mixed solution | 1.0 mL |
| Distilled water | 1 L |

**Table 2**

| Composition of Metal solution | |
|---|---|
| Ferric chloride hexahydrate | 0.5 g |
| Manganese chloride tetrahydrate | 0.5 g |
| Sodium molybudate dihydrate | 0.1 g |
| Cupric chloride dihydrate | 0.05 g |
| Sodium tungstate dihydrate | 0.05 g |
| Hydrochloric acid | 10 mL |
| Distilled water | 1 L |

**Table 3**

| Vitamin mixed solution | |
|---|---|
| Potassium pantothenate | 400 mg |
| Inositol | 200 mg |
| Nicotinic acid | 400 mg |
| Piridoxine hydrochloride | 400 mg |
| p-Amino benzoic acid | 200 mg |
| Cyanocobalamine | 0.5 mg |
| Riboflavin | 200 mg |
| Folic acid | 200 mg |
| Distilled water | 1 L |

### Method by culture

Culture liquid was sterilized by heating at 120°C for 15minutes, then 1 g of 5-sulfoisophthalic acid sodium salt was added. *Ochrobactrum anthropi* S9 was inoculated thereto and was subjected to shaking culture at 28°C for 4 days. It was observed that 1.19 mM of 5-hidroxyisophthalic acid (218 mg/L) was accumulated.

### Example 2

Treatment was conducted in the same manner as in Example 1 except that the amount of sodium glutamate was changed to 10.0 g. It was observed that 1.00 mM of 5-hydroxyisophthalic acid (182 mg/L) was accumulated.

### Method by bringing the microorganisms into contact with the substrate

### Example 3

To the culture medium similar to that used in Example 1, *Ochrobactrum anthropi* S9 was inoculated and subjected to shaking culture at 28°C for 60 hours. The resulting culture liquid (100 mL) was subjected to a centrifugal separation (12,000 rpm, 20 min.), subsequently, the resulting microorganisms were suspended in 6.7 mL of a 0.85% aqueous sodium chloride solution. It was mixed with 6.7 mL of a reaction solution containing 4.5 mM of 5-sulfoisophthalic acid, 0.1 M glycine-sodium hydroxide buffer (pH 9.5) and 5% (w/v) glucose and subjected to shaking at 30°C for 24 hours. It was observed that 1.85 mM of 5-hydroxyisophthalic acid (328 mg/L) was accumulated.

### Example 4

To the culture medium similar to that used in Example 1, *Ochrobactrum anthropi* S9 was inoculated and was subjected to shaking culture at 1158 rpm at a temperature of 33°C for 48 hours.

The obtained culture liquid was subjected to a centrifugal separation (4°C, 8,000 rpm, 15 minutes), and then, the resulting microorganisms were washed with physiological saline for two times. They were suspended in physiological saline so that the final volume became one-fifteenth of the volume of the culture liquid.

0.88 ml of the obtained suspension of resting microorganisms was diluted with a reaction solution containing 15 mM of 5-sulfoisophthalic acid solution, 0.1 M glycine-sodium hydroxide buffer (pH 9.5) and 0.4 M glucose solution to prepare a reaction mixture so that the total volume of the liquid became 4 mL.

The reaction mixture was subjected to shaking at 175 strokes/min at a temperature of 25°C, and after 24, 36 and 48 hours, 100 µM of glycine and 200 µM of glucose were added to the reaction mixture. After 60 hours, the reaction was terminated by a centrifugal separation (12,000 rpm, 5 minutes). A supernatant was analyzed by HPLC, and it was observed that 2.75 mM of 5-hydroxyisophthalic acid (501 mg/L) was accumulated.

## Claims

1. A microbial transformation process for producing a hydroxyaromatic compound represented by the formula (II): 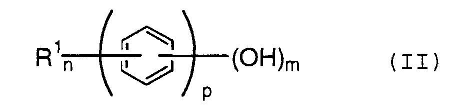 wherein R¹ represents a carboxyl group, a C₂₋₅ alkoxycarbonyl group, a hydroxyl group, a nitro group, a cyano group or a C₁₋₅ alkyl group, m represents an integer of 1 to 5, n represents an integer of 0 to 5, p represents an integer of 1 to 3, m, n and p satisfy a relation of m+n≦4+2p, and when n is 2 or more, R¹ may be the same or different from each other,
which comprises bringing an aromatic sulfonic acid compound represented by the formula (I): 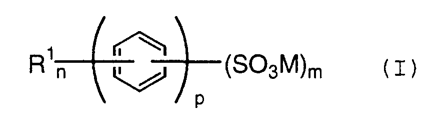 wherein each of R¹, m, n and p has the same meaning as defined above, and M represents a hydrogen atom, an alkali metal, an alkaline earth metal or a C₁₋₅ alkyl group,
in contact with a culture medium, cells or treated cells of a microorganism belonging to *Ochrobactrum* genus.

2. The process for producing a hydroxyaromatic compound according to Claim 1, wherein R¹ is a carboxyl group or a C₂₋₅ alkoxycarbonyl group, and p is 1.

3. The process for producing a hydroxyaromatic compound according to Claim 2, wherein n is 2 and m is 1.

4. The process for producing a hydroxyaromatic compound according to Claim 1, wherein the aromatic sulfonic acid compound represented by the formula (I) is a compound selected from the group consisting of benzenesulfonic acid sodium salt, 1-naphthalenesulfonic acid sodium salt and 5-sulfonaphthalic acid sodium salt.

5. The process for producing a hydroxyaromatic compound according to Claim 3, wherein the compound represented by the formula (I) is 5-sulfoisophthalic acid sodium salt and the compound represented by the formula (II) is 5-hydroxyisophthalic acid.

6. The process for producing a hydroxyaromatic compound according to any one of Claims 1 to 4, wherein the microorganism belonging to *Ochrobactrum* genus is *Ochrobactrum anthropi.*

7. The process for producing a hydroxyaromatic compound according to Claim 6, wherein *Ochrobactrum anthropi* is *Ochrobactrum anthropi* S9 deposited under a receipt number of FERM BP-7546.

8. The process for producing a hydroxyaromatic compound according to Claim 1, wherein the process is carried out by
(a) a method wherein microorganisms are cultured in a culture medium containing the compound represented by the formula (1) as a substrate;
(b) a method wherein the microorganisms are collected from a culture medium where they were cultured and brought into contact with the compound represented by the formula (1); or
(c) a method wherein a cell-free extract prepared from the cells of the microorganisms are brought into contact with the compound represented by the formula (1).

9. The process for producing a hydroxyaromatic compound according to Claim 8, wherein culture is conducted under an aerobic condition by using an aerobic culture method at a temperature in the range of 20 to 30°C.

10. The process for producing a hydroxyaromatic compound according to Claim 8, wherein the method (a) is carried out by culturing the microorganisms with addition of the compound represented by the formula (1) by adding at a point where an hydroxylation activity of transforming microorganisms starts to elevate.

11. The process for producing a hydroxyaromatic compound according to Claim 8, wherein the compound represented by the formula (1) is added 1 to 5 days after the start of culturing the transforming microorganisms in an amount in the range of 0.01 to 5.0% based on the medium.

12. The process for producing a hydroxyaromatic compound according to Claim 11, wherein the amount of the compound represented by the formula (1) is in the range of 0.025 to 0.5% based on the medium.

13. The process for producing a hydroxyaromatic compound according to Claim 8, wherein the method (b) is carried out by culturing transforming microorganisms in the presence of a little amount of a substrate until an hydroxylation activity of the transforming microorganisms is maximized.

14. The process for producing a hydroxyaromatic compound according to Claim 13, wherein culture is carried out for 2 or 3 days after the start of culture, and then, the transforming microorganisms are brought into contact with a starting compound in an aqueous solvent of a 10 to 100 mM phosphate buffer of pH 5 to 9 at a temperature of 20 to 33°C and a concentration of the substrate in the range of 0.01 to 5.0% based on the culture medium for 1 to 8 days.

15. The process for producing a hydroxyaromatic compound according to Claim 8, wherein the cell-free extract used in the method (c) is obtained by applying a physical or chemical means to the microorganisms to give a product in a form of crushed cells by trituration or ultrasonic treatment, or in a form of a dissolved solution of the cells by treating with an organic solvent, a surfactant or an enzyme.

16. *Ochrobactrum anthropi* S9 which is deposited under a receipt number of FERM BP-7546.
